# FASCICULE DE BREVET EUROPEEN

(11) **EP 1 241 996 B1**
(45) Date de publication et mention de la délivrance du brevet: **10.09.2008**
(21) Numéro de dépôt: 00946048.6
(22) Date de dépôt: 30.06.2000
(51) Int. Cl.: A61B 17/88

(54) **INSTRUMENT POUR LA MISE EN PLACE D'UNE VIS PEDICULAIRE**
GERÄT ZUR POSITIONIERUNG EINER PEDIKELSCHRAUBE
INSTRUMENT FOR SETTING A PEDICLE SCREW

(30) Priorité: 01.07.1999 FR 9908497
(43) Date de publication de la demande: 25.09.2002
(73) Titulaire: Spinevision S.A., 75012 Paris (FR)
(72) Inventeur: Bolger, Ciaran, Frampton Cotterel, Bristol BS 362 NE (GB); Bolger, John, Rath Farnham, Dublin 16 (IE)
(74) Mandataire: Breese, Pierre
(86) Numéro de dépôt international: PCT/FR2000/001871
(87) Numéro de publication internationale: WO 2001/001875

(56) Documents cités:
- US-A- 4 824 433
- US-A- 5 196 015
- US-A- 5 284 153
- US-A- 5 779 642

## Description

La présente invention concerne le domaine de chirurgie du rachis, et plus précisément le domaine du forage du pédicule vertébral, notamment pour la pose d'une vis pédiculaire.

Il est connu d'utiliser des instruments de forage manuels ou motorisés, rotatifs ou mobiles alternativement autour d'une position médiane.

On connaît notamment le brevet européen EP287823 décrivant un instrument chirurgical à usage manuel, en particulier pour ostéosynthèse, muni d'un manche auquel est fixé un outil, tel qu'une pointe de tournevis, un taraud, d'un arbre supporté en rotation de façon coaxiale dans le manche et sur lequel sont fixées deux roues dentées à distance l'une de l'autre, d'un cliquet d'arrêt disposé dans le manche et actionnable de l'extérieur, et qui dans une position de fin de course n'engrène que dans la couronne dentée de l'une des roues dentées et qui dans l'autre position de fin de course n'engrène que dans la couronne dentée de l'autre roue dentée.

Le problème qui se pose avec les instruments de l'état de la technique est celui du guidage de l'instrument de forage, et plus précisément du choix du point d'entrée et de l'orientation de l'outil de forage, afin d'éviter tout risque de sortie du pédicule.

La difficulté vient de la nécessité de traverser l'os spongieux en restant en contact avec la corticale, en évitant impérativement une trajectoire trop latérale susceptible de léser la moelle épinière ou les nerfs rachidiens.

La difficulté est encore augmentée lorsque le pédicule présente des déformations imposant une sortie de l'instrument de forage à un niveau du pédicule pour rentrer après la déformation dans le pédicule sans léser des parties innervées.

Des études cliniques font apparaître que ce problème ne trouve pas à ce jour de solution satisfaisante, et il semble que 25% des vis pédiculaires soient mal positionnées.

Pour répondre à ce problème, l'invention consiste à détecter un nerf par l'électrification de l'instrument de forage

Certes, on connaît dans l'art antérieur des documents concernant la détection de réponse musculaire par analyse d'un signal électrique. Mais la finalité de ces équipements est la surveillance de l'activité nerveuse, en particulier pendant une anesthésie.

On connaît par exemple le brevet américain US5,284,153. Ce document divulgue et revendique un procédé de localisation d'un nerf. Ce document propose plusieurs étapes comprenant la détermination de la distance entre le nerf et le moyen de stimulation. Cette étape n'a aucun intérêt pour apporter une réponse au problème du guidage d'un instrument de forage d'un pédicule vertébral.

Le brevet américain US5, 779, 642 divulgue un procédé de localisation d'un nerf pour le guidage d'une aiguille de ponction, mettant en oeuvre une étape de localisation de l'aiguille par un moyen ultrason. Le brevet US4,824,433 concerne également une application pour le positionnement d'une aiguille de ponction, par l'utilisation d'un courant électrique alimentant l'aiguille et la détection de ce signal.

Le brevet US4,962,766 divulgue un équipement de localisation de nerfs pendant une intervention chirurgicale. Le but est l'étude de la fonction du nerf.

Aucun de ces document ne concerne expressément, ni ne suggère, le guidage d'un instrument de forage d'un pédicule vertébral.

Les caractéristiques du préambule de la revendication 1 sont connues du document US-A-5196015. Ce document concerne un procédé d'insertion d'une vis pédiculaire, selon lequel on détecte le potentiel évoqué lors de l'insertion d'une vis pédiculaire. La détection de la réaction du patient est réalisée par une observation visuelle, ce qui ne permet pas de garantir une réaction suffisamment rapide de l'opérateur. En outre, le seuil de déclenchement de la réaction est dépendante du patient, de sa condition, et ne permet pas de garantir une fiabilité et une sensibilité satisfaisante de la détection.

L'invention concerne dans son acception la plus générale un équipement de forage du pédicule vertébral, notamment pour la mise en place d'une vis pédiculaire, selon la revendication 1.

Selon une variante, le moyen d'alerte est un signal sonore ou lumineux.

Selon une autre variante, on modifie le point d'entrée de l'instrument de forage en cas de déclenchement du moyen d'alerte.

Selon un mode de réalisation préféré, on modifie l'orientation de l'instrument de forage en cas de déclenchement du moyen d'alerte.

Selon un mode de mise en oeuvre particulier, la source de tension raccordée à l'instrument de forage délivre un signal électrique continu ou des impulsions présentant une période inférieure à 5 hertz.

Avantageusement, l'équipement de forage comprend un outil de forage, une aiguille implantable et un dispositif de commande comprenant un générateur de tension dont la sortie est connectable à l'outil de forage, et un circuit de détection dont la sortie est connectable à l'aiguille implantable.

L'invention concerne encore une aiguille implantable et des cordons de liaison stérile pour la mise en oeuvre du procédé selon l'invention.

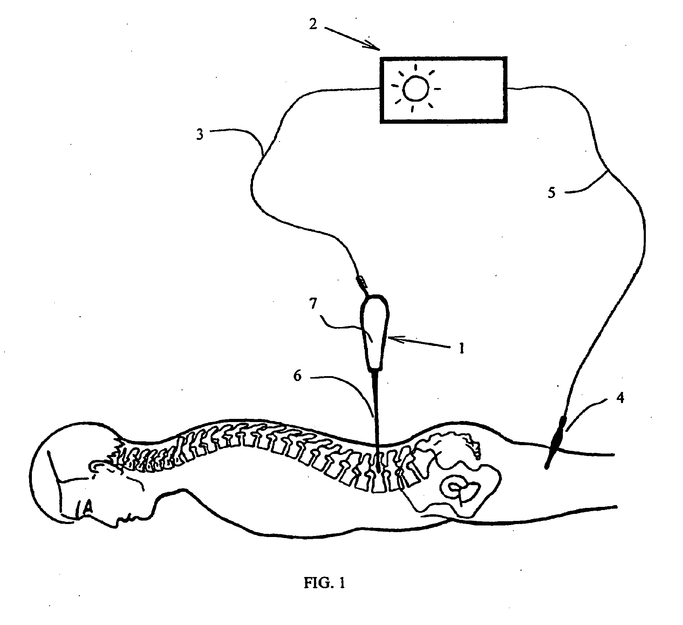

L'invention sera mieux comprise à la lecture de la description qui suit, se référant aux dessins annexés où la figure 1 représente une vue schématique de l'équipement selon l'invention.

L'équipement selon l'exemple non limitatif de réalisation de l'invention comprend un instrument de forage (1) relié à un boîtier de commande (2) par l'intermédiaire d'un cordon électrique (3) stérile. Le boîtier de commande (2) est par ailleurs relié à une aiguille (4), fine, par l'intermédiaire d'un autre cordon électrique (5) stérile.

L'instrument de forage (1) comporte une mèche ou un foret métallique formant un outil de forage (6) solidaire d'un manche ou d'un moteur (7). La liaison entre le cordon électrique (3) et l'outil de forage (6) est réalisée par un connecteur électrique tournant ou par un mandrin en un matériau conducteur et une borne de liaison prévue sur le manche ou la motorisation de l'outil de forage.

Le boîtier (2) délivre une tension électrique continue de faible courant et de faible tension. Ce signal est continu ou haché, avec une période inférieure à 5 hertz et de préférence inférieure à 1 hertz, afin de permettre une mise en corrélation du signal détecté par l'aiguille (4) et le signal appliqué à l'outil de forage (6) et supprimer les signaux parasites.

L'aiguille (4) est implantée dans un muscle adéquat de la jambe du patient. Le circuit électronique du boîtier (2) comporte un détecteur synchrone, le cas échéant, avec le générateur de tension. La détection d'un signal électrique par l'aiguille (4) provoque l'émission d'un signal sonore et/ou visuel, avertissant le chirurgien d'un contact entre l'instrument de forage (1) et un nerf.

Le chirurgien peut ainsi modifier l'orientation de l'instrument de forage, et éventuellement retirer l'instrument de forage (1) pour engager l'outil de forage (6) en un point d'entrée décalé.

## Revendications

1. Equipement de forage du pédicule vertébral notamment pour la mise en place d'une vis pédiculaire à l'aide d'un outil de forage (6) manuel ou motorisé, ledit équipement comportant un instrument de forage (1) comportant l'outil de forage (6), une source de tension et des moyens de raccordement de l'instrument de forage (1) à la source de tension, **caractérisé en ce qu'**il comporte une électrode de détection (4) d'un signal électrique et un moyen produisant une alerte, en cas de détection par l'électrode implantée, d'un signal corrélé avec la source de tension raccordée à l'instrument de forage (1).

2. Equipement de forage du pédicule vertébral selon la revendication 1, **caractérisé en ce que** le moyen d'alerte est un signal sonore ou lumineux.

3. Equipement de forage du pédicule vertébral selon la revendication 1 ou 2, **caractérisé en ce que** la source de tension raccordée à l'instrument de forage (1) délivre un signal électrique continu ou des impulsions présentant une période inférieure à 5 hertz.

4. Equipement de forage du pédicule vertébral selon l'une quelconque des revendications 1 à 3, **caractérisé en ce que** la source de tension est raccordée à l'instrument de forage (1) à l'aide d'un cordon électrique (3).

5. Equipement de forage du pédicule vertébral selon l'une quelconque des revendications 1 à 4, **caractérisé en ce que** l'électrode est raccordée à l'instrument de forage (1) à l'aide d'un cordon électrique (5).

6. Equipement de forage du pédicule vertébral selon la revendication 5, **caractérisé en ce que** l'électrode est une aiguille implantable (4) comportant des moyens pour être connectée audit cordon électrique (5) et **en ce qu'**elle est apte à être implantée dans un muscle de la jambe d'un patient.

7. Equipement de forage du pédicule vertébral selon la revendication 6, **caractérisé en ce qu'**il comprend un dispositif de commande comprenant un générateur de tension dont la sortie est connectable à l'outil de forage (6) et un circuit de détection d'un signal électrique dont la sortie est connectable à l'aiguille implantable (4).

8. Equipement de forage du pédicule vertébral selon la revendication 4, **caractérisé en ce que** la liaison entre le cordon électrique (3) et l'instrument de forage est réalisée par un connecteur rotatif.

## Claims

1. An equipment for drilling the pedicle of arch of vertebra, more particularly for positioning a pedicle screw using a manual or powered drilling tool (6), said equipment including a drilling instrument (1) including the drilling tool (6), a voltage source and means for connecting the drilling instrument (1) to the voltage source, **characterized in that** it includes an electric signal detection electrode (4) and means delivering a warning, when the implanted electrode detects a signal correlated to the voltage source connected to the drilling instrument (1).

2. An equipment for drilling the pedicle of arch of vertebra according to claim 1, **characterized in that** the warning means is a sound or light signal.

3. An equipment for drilling the pedicle of arch of vertebra according to claim 1 or 2, **characterized in that** the voltage source connected to the drilling instrument (1) delivers a continuous electric signal or pulses having a period of less than 5 hertz.

4. An equipment for drilling the pedicle of arch of vertebra according to claims 1 to 3, **characterized in that** the voltage source is connected to the drilling instrument (1) by means of an electric cord (3).

5. An equipment for drilling the pedicle of arch of vertebra according to claims 1 to 4, **characterized in that** the electrode is connected to the drilling instrument (1) by means of an electric cord (5).

6. An equipment for drilling the pedicle of arch of vertebra according to claim 5, **characterized in that** the electrode is an implantable needle (4) including means for connection to said electric cord (5) and **in that** it is able to be implanted in a muscle of a patient's leg.

7. An equipment for drilling the pedicle of arch of vertebra according to claim 6, **characterized in that** it comprises a control device comprising a voltage generator, the output of which can be connected to the drilling tool (6) and an electric signal detection circuit, the output of which can be connected to the implantable needle (4).

8. An equipment for drilling the pedicle of arch of vertebra according to claim 4, **characterized in that** the connection between the electric cord (3) and the drilling instrument is through a rotating connector.

## Patentansprüche

1. Ausrüstung zum Bohren der Wirbelbogenwurzel insbesondere zum Anbringen einer Wirbelbogenwurzelschraube mit Hilfe eines manuellen oder motorbetriebenen Bohrwerkzeugs (6), wobei die besagte Ausrüstung ein Bohrinstrument (1) umfaßt, das das Bohrwerkzug (6), eine Spannungsquelle und Mittel zum Anschließen des Bohrinstruments (1) an die Spannungsquelle umfaßt, **dadurch gekennzeichnet, daß** sie eine Elektrode zum Entdecken (4) eines elektrischen Signals und ein Mittel umfaßt, das eine Warnung erzeugt, wenn die eingebaute Elektrode ein Signal entdeckt, das mit der an das Bohrinstrument (1) angeschlossenen Spannungsquelle korreliert.

2. Ausrüstung zum Bohren der Wirbelbogenwurzel nach Anspruch 1, **dadurch gekennzeichnet, daß** das Warnungsmittel ein akustisches oder Lichtsignal ist.

3. Ausrüstung zum Bohren der Wirbelbogenwurzel nach Anspruch 1 oder 2, **dadurch gekennzeichnet, daß** die an das Bohrinstrument (1) angeschlossene Spannungsquelle ein kontinuierliches elektrisches Signal oder Impulse mit einer Periode von unter 5 Hertz abgibt.

4. Ausrüstung zum Bohren der Wirbelbogenwurzel nach einem beliebigen der vorstehenden Ansprüche 1 bis 3, **dadurch gekennzeichnet, daß** die Spannungsquelle mit einem Stromkabel (3) an das Bohrinstrument (1) angeschlossen ist.

5. Ausrüstung zum Bohren der Wirbelbogenwurzel nach einem beliebigen der vorstehenden Ansprüche 1 bis 4, **dadurch gekennzeichnet, daß** die Elektrode mit einem Stromkabel (5) an das Bohrinstrument (1) angeschlossen ist.

6. Ausrüstung zum Bohren der Wirbelbogenwurzel nach Anspruch 5, **dadurch gekennzeichnet, daß** die Elektrode eine einbaubare Nadel (4) ist, die Mittel umfaßt, damit sie an das besagte Stromkabel (5) angeschlossen werden kann, und **dadurch**, daß sie geeignet ist, in einen Muskel des Beins eines Patienten eingebaut zu werden.

7. Ausrüstung zum Bohren der Wirbelbogenwurzel nach Anspruch 6, **dadurch gekennzeichnet, daß** sie eine Steuervorrichtung umfaßt, die einen Spannungsgenerator umfaßt, dessen Ausgang an das Bohrwerkzeug (6) anschlußfähig ist, und einen Entdeckungskreis für ein elektrisches Signal, dessen Ausgang an die einbaufähige Nadel (4) anschlußfähig ist.

8. Ausrüstung zum Bohren der Wirbelbogenwurzel nach Anspruch 4, **dadurch gekennzeichnet, daß** die Verbindung zwischen dem Stromkabel (3) und dem Bohrinstrument durch einen Drehverbinder ausgeführt ist.
